(19) **Europäisches Patentamt / European Patent Office / Office européen des brevets**

(11) **EP 1 581 274 B1**

(12) **FASCICULE DE BREVET EUROPEEN**

(45) Date de publication et mention de la délivrance du brevet:
**05.08.2009 Bulletin 2009/32**

(51) Int Cl.:
***A61L 27/26*** *(2006.01)*     ***A61K 47/30*** *(2006.01)*

(21) Numéro de dépôt: **04700134.2**

(22) Date de dépôt: **05.01.2004**

(86) Numéro de dépôt international:
**PCT/FR2004/000003**

(87) Numéro de publication internationale:
**WO 2004/069294 (19.08.2004 Gazette 2004/34)**

(54) **COMPOSITIONS INJECTABLES GELIFIANTES A BASE DE PARTICULES DE POLYMERES RETICULES ET DE POLYMERES NON RETICULES, ET LEURS APPLICATIONS**

INJIZIERBARE GELBILDENDE ZUSAMMENSETZUNGENAUF BASIS VON VERNETZTEN UND UNVERNETZTEN POLYMEREN UND IHRE VERWENDUNG

INJECTABLE GEL-FORMING COMPOSITIONS BASED ON CROSS-LINKED AND NON-CROSS-LINKED POLYMERS AND THE USE THEREOF

(84) Etats contractants désignés:
**AT BE BG CH CY CZ DE DK EE ES FI FR GB GR HU IE IT LI LU MC NL PT RO SE SI SK TR**

(30) Priorité: **06.01.2003 FR 0300067**

(43) Date de publication de la demande:
**05.10.2005 Bulletin 2005/40**

(73) Titulaires:
• **ASSISTANCE PUBLIQUE, HOPITAUX DE PARIS**
**75004 Paris (FR)**
• **UNIVERSITE PARIS-SUD (PARIS XI)**
**91405 Orsay Cédex (FR)**

(72) Inventeurs:
• **LAURENT, Alexandre**
**Courbevoie 92400 (FR)**
• **LABARRE, Denis**
**Villebon 91140 (FR)**
• **LABSKY, Jiri**
**102 00 Praha 6 (CZ)**
• **HONIGER, Jiri**
**F-94800 Villejuif (FR)**

• **CHAPOT, René**
**45117 Essen Rüttenscheid (DE)**
• **WASSEF, Michel**
**F-75005 Paris (FR)**
• **SERON, Aymeric**
**F-75010 Paris (FR)**

(74) Mandataire: **Goulard, Sophie et al**
**Cabinet ORES**
**36 Rue de St Pétersbourg**
**75008 Paris Cedex (FR)**

(56) Documents cités:
**EP-A- 0 291 177**      **EP-A- 0 466 300**
**EP-A- 0 826 381**

• **BAJAJ, P. ET AL: "Studies on acrylonitrile-hydroxyalkyl-methacrylate copolymer fibers" TEXTILE RESEARCH JOURNAL (1980), 50(4), 218-23, 1980, XP009014272**
• **BAJAJ, P. ET AL: "Copolymerization of acrylonitrile with 2-hydroxyalkyl methacrylates" JOURNAL OF POLYMER SCIENCE, POLYMER CHEMISTRY EDITION (1979), 17(2), 595-602, 1979, XP009014275**

## Description

La présente Invention est relative à des compositions injectables gélifiantes renfermant une association de polymères linéaires et de polymères réticulés, ainsi qu'à leurs utilisations, notamment pour la réalisation d'occlusions thérapeutiques (embolisations), le comblement de conduits et cavités ou l'implantation par voie percutanée.

L'utilisation de biomatériaux résorbables ou non résorbables, est fréquente dans le milieu médical. Ces biomatériaux peuvent se présenter sous diverses formes et être utilisés par exemple pour la réalisation d'occlusions vasculaires thérapeutiques (embolisations), pour la reconstruction tissulaire, le traitement du reflux gastro-oesophagien, de l'incontinence urinaire, l'implantation percutanée, la réduction des rides et en général le comblement de conduits et cavités.

Dans ce contexte, il est courant d'utiliser des matériaux injectables se présentant sous forme gélifiée et qui se solidifient *in situ* juste après l'injection.

C'est ainsi qu'il a déjà été proposé des colles et ciments acryliques injectables, par exemple à base d'isobutylcyanoacrylate, de N-butylcyanoacrylate ou encore de poly(méthacrylate de méthyle) (PMMA). Ces matériaux présentent toutefois l'inconvénient d'être toxiques et difficiles à manipuler dans la mesure où ils peuvent entraîner l'encollage du cathéter utilisé pour l'injection. Il est de plus difficile de maîtriser le temps de polymérisation *in situ* de ces matériaux.

Il a également déjà été proposé de remplacer les colles et ciments acryliques, notamment dans les brevets US 5,580,568, US 5,695,480 et US 5,851,508, par des solutions gélifiantes composées principalement d'un polymère insoluble dans l'eau en solution dans un solvant biocompatible et miscible à l'eau tel que par exemple le diméthylsulfoxyde (DMSO). Selon ces documents, le polymère en solution se solidifie *in situ* après injection selon un phénomène d'extraction du solvant qui est miscible à l'eau et par conséquent miscible avec le milieu physiologique naturellement aqueux.

Cependant, ces solutions gélifiantes présentent un certain nombre d'inconvénients :

- le temps nécessaire à la sortie du solvant conditionne le temps de gélification du polymère. Ainsi, plus la quantité de solvant présente dans la solution est importante, plus le temps de gélification est long ;
- la solution en cours de gélification *in situ* ne possède qu'une faible résistance mécanique face au flux circulant. C'est la couche externe en contact avec le sang qui se gélifie en premier, tandis que le coeur du dépôt reste liquide. La déformabilité du dépôt sous flux reste importante tant que la phase de gélification du polymère n'est pas achevée. Ce temps peut être trop long pour qu'il y ait solidification du polymère avant que la solution ait été emportée par le flux sanguin. En pratique, l'utilisation de telles solutions gélifiantes impose une injection très lente afin que la solution puisse gélifier *in situ* au fur et à mesure qu'elle sort du cathéter. Si l'injection n'est pas réalisée de façon suffisamment lente, la solution gélifiante se déforme pour être soit étirée et emportée par le flux sanguin comme un liquide visqueux, soit plaquée contre la paroi vasculaire en couche plus ou moins épaisse ;
- lors de la solidification du polymère, il se produit une diminution de volume de l'ordre de 20 à 80 % (phénomène de retrait), qui est dû à l'extraction du solvant par le flux circulant et qui est peu compensée par l'entrée du liquide physiologique dans le polymère en cours de solidification (précipitation), ce qui entraîne une moindre dépôt de polymère que de solution injectée. Il n'est pas possible de compenser cette perte de volume par une augmentation de la concentration en polymère au sein de la solution gélifiante car cela entraînerait une augmentation importante de la viscosité de la solution gélifiante qui gênerait ou empêcherait l'injection au travers d'aiguilles ou de microcathéters par exemple ;
- les solvants biocompatibles miscibles à l'eau utilisés pour solubiliser les polymères, en particulier le DMSO, ont une toxicité vasculaire locale et systémique importante (Mottu F et al., PDA J. Pharm. Sci. Technol., 2000, 54(6), 456-469), qui est bien évidemment proportionnelle à la dose relarguée dans le flux sanguin. De plus, avec un tel solvant, l'injection des solutions gélifiantes requiert l'utilisation de cathéters spéciaux, conçus pour résister au solvant. Cependant, même dans ce cas, le solvant peut néanmoins endommager les microcathéters par lesquels il est injecté.

C'est afin de remédier à l'ensemble de ces problèmes que les Inventeurs ont mis au point ce qui fait l'objet de l'Invention.

La présente Invention a donc pour objet une composition injectable gélifiante à base de polymères pour l'implantation intra-tissulaire et/ou intra-vasculaire, caractérisée par le fait qu'elle comprend :

- au moins un polymère linéaire insoluble dans l'eau et soluble dans au moins un solvant miscible à l'eau,
- au moins un polymère réticulé hydrophile et insoluble dans l'eau, ledit polymère réticulé ayant une affinité pour ledit polymère linéaire, et
- au moins un solvant biocompatible miscible à l'eau ; et par le fait qu'elle se présente sous la forme d'une suspension de particules dudit polymère réticulé hydrophile dans une solution dudit polymère linéaire.

La composition injectable gélifiante conforme à l'Invention présente les avantages et caractéristiques physico-chimiques suivants :

- elle permet d'obtenir, pour un même volume de composition gélifiante injecté qu'avec les solutions gélifiantes décrites dans l'état antérieur de la technique, une quantité finale de polymère déposée comparativement plus importante par unité de volume injectée ;
- elle présente une viscosité inférieure à celle des solutions injectables décrites dans l'état antérieur de la technique contenant la même masse de polymère soluble ;
- elle conduit à une cohésion plus forte du gel déposé dans la lumière vasculaire ;
- elle permet de diminuer la quantité de solvant injectée au patient ;
- elle permet de raccourcir le temps de solidification du polymère linéaire par rapport à une solution gélifiante classique, car, la quantité de solvant étant moindre, la cinétique de sortie du solvant du mélange est plus rapide.

Lorsque la composition injectable gélifiante conforme à l'Invention est injectée dans un liquide physiologique, qui par nature est aqueux, le solvant quitte le mélange de polymères, ce qui entraîne la précipitation et la solidification du polymère linéaire qui emprisonne alors le polymère réticulé hydrophile. La quantité totale et le volume de matière sont augmentés par la présence du polymère réticulé hydrophile qui, du fait de son affinité pour l'eau, gonfle en milieu aqueux.

Au sens de la présente Invention, le terme "affinité" utilisé pour qualifier le polymère réticulé présent au sein de la composition injectable gélifiante s'entend comme toute cause qui sollicite le polymère réticulé à se combiner au polymère linéaire et qui les maintient unis quand la combinaison est réalisée. A titre d'exemple, cette affinité peut notamment être chimique.

Selon l'Invention, le ou les polymères linéaires sont de préférence choisis parmi les polymères neutres ou peu chargés.

Parmi de tels polymères, on peut notamment citer les poly(acrylates d'alkyle), les poly(méthacrylates d'alkyle), les poly(cyanoacrylates d'alkyle), les poly(acétate de vinyle), les poly(butyrate de vinyle), les poly(vinyle formal), les poly (vinyle acétal), les poly(vinyle butyral), les poly(oxypropylène), les poly(oxytétraméthylène), les esters de cellulose insolubles dans l'eau, les esters de chitosane ou autres polysaccharides insolubles dans l'eau, les polylactides, les polyglycolides, les polycaprolactone, les esters de poly(acide malique), les esters de poly(acide maléique), les esters de poly(acide fumarique), et les copolymères ou dérivés linéaires insolubles dans l'eau comprenant ces composés.

Parmi ces polymères, on peut tout particulièrement citer le polyhydroxyéthylméthacrylate (p(HEMA)), le poly (méthacrylate de méthyle) (PMMA), le polyhydroxypropylméthacrylate p(HPMA), les copolymères d'hydroxyéthylméthacrylate ou d'hydroxypropylméthacrylate et d'acrylonitrile (HEMA-AN ou HPMA-AN), les copolymères d'hydroxyéthylméthacrylate ou d'hydroxypropylméthacrylate et de N-*ter*-butylacrylamide (HEMA-TBA ou HPMA-TBA), les copolymères d'hydroxyéthylméthacrylate ou d'hydroxypropylméthacrylate et d'acétoacétoxyéthylméthacrylate (HEMA-AAMA ou HPMA-AAMA), le poly-(N-acryloyl-2-amino-2-hydroxyméthyl-1,3-propanediol) tel que le produit vendu sous la dénomination commerciale TRISACRYL® par la société BIOSEPRA (France), le poly-(n-2-hydroxypropyl méthacrylamide), et leurs dérivés.

Selon une forme de réalisation préférée de l'Invention, le ou les polymères linéaires sont de préférence choisis parmi les copolymères d'hydroxypropylméthacrylate et d'acrylonitrile (HPMA-AN), les copolymères d'hydroxypropylméthacrylate et de N-*ter*-butylacrylamide (HPMA-TBA) et les copolymères d'hydroxypropylméthacrylate et d'acétoacétoxyéthylméthacrylate (HPMA-AAMA).

Le ou les polymères linéaires représentent de préférence de 3 à 25 % (m/V) de la composition injectable gélifiante conforme à l'Invention et encore plus préférentiellement de 5 à 20 % (m/V).

Selon l'Invention, le ou les polymères réticulés hydrophiles peuvent notamment être choisis parmi les polymères issus de la réticulation des polymères linéaires insolubles dans l'eau tels que décrits ci-dessus.

Le ou les polymères réticulés hydrophiles peuvent également être choisis parmi les polymères issus de la réticulation de polymères linéaires solubles dans l'eau tels que les alginates ; les dérivés de l'amidon ; les éthers de cellulose ; les acétates de cellulose de degré de substitution compris entre 0,6 et 0,8 ; les sulfates de cellulose ; les polysaccharides hydrosolubles tels que les dextranes ; les sels de chitosanes ; les polymères acryliques et méthacryliques ; les polyacrylamides et polyméthacrylamides substitués ou non ; les dérivés hydrolysés des poly(vinylacétates) tels que les poly(vinylalcools) ; les polymères dérivés du poly(oxyéthylène), la poly(éthylèneimine) ; les sels solubles de la poly(vinylpyridine) ; la poly(vinylpyrrolidone) ; les polyuréthanes ; leurs sels et leurs copolymères.

Parmi les polymères réticulés, on peut tout particulièrement citer les polymères réticulés de HEMA, de HPMA ou de poly-(N-acryloyl-2-amino-2-hydroxyméthyl-1,3-propanediol), ainsi que les copolymères réticulés de HEMA et de poly-(N-acryloyl-2-amino-2-hydroxyméthyl-1,3-propanediol) ou de HPMA et de poly-(N-acryloyl-2-amino-2-hydroxyméthyl-1,3-propanediol).

La réticulation des polymères peut être effectuée de façon classique selon toute méthode connue de l'homme du métier, à l'aide d'un agent de réticulation tel que par exemple le méthylène bis acrylamide.

Le taux de réticulation du polymère réticulé est de préférence compris entre 0,5 et 12 % (m/V) et encore plus préférentiellement entre 1 et 5 % (m/V).

Le ou les polymères réticulés représentent de préférence de 1 à 30 % (m/V) de la composition injectable gélifiante conforme à l'invention et encore plus préférentiellement de 8 à 12 % (m/V).

Selon l'Invention, la taille des particules de polymère réticulé peut varier entre 1 et 1000 $\mu$m et de préférence entre 20 et 100 $\mu$m.

Selon une forme de réalisation préférée de l'Invention, la composition injectable gélifiante comprend un polymère linéaire et un polymère réticulé de même nature, sous forme de particules. A titre d'exemple, la composition injectable gélifiante conforme à l'Invention peut renfermer au moins un polymère linéaire de HEMA ou de HPMA ou un copolymère linéaire à base de HEMA ou de HPMA et des particules de polymères réticulés de HEMA, de HPMA ou de poly-(N-acryloyl-2-amino-2-hydroxyméthyl-1,3-propanediol), et/ou de copolymères réticulés de HEMA et de poly-(N-acryloyl-2-amino-2-hydroxyméthyl-1,3-propanediol) ou de HPMA et de poly-(N-acryloyl-2-amino-2-hydroxyméthyl-1.3-propanediol).

Une composition injectable gélifiante particulièrement préférée selon l'Invention comprend :

- au moins un copolymère linéaire à base de HEMA ou de HPMA, et
- au moins des particules de copolymères de HEMA ou de HPMA et de poly-(N-acryloyl-2-amino-2-hydroxyméthyl-1,3-propanediol) réticulés.

Le ou les solvants biocompatibles miscibles à l'eau sont de préférence choisis parmi les solvants organiques faiblement visqueux afin de permettre leur administration par des aiguilles ou des cathéters de diamètre égal ou inférieur au millimètre.

Selon l'Invention, on entend par biocompatible, tout solvant compatible d'un point de vue pharmaceutique (Food and Drug Administration (FDA), Norme ISO 10993-13 (1998)) pour être injecté chez l'homme ou l'animal.

Parmi de tels solvants, on peut en particulier citer la N-méthyl-pyrrolidone, le diméthyléthylamide, le diméthyléther de diéthylèneglycol (Diglyme®), l'éthyl-lactate, l'éthanol, le diméthoxyéthane, le DMSO, le glycofurol et leurs mélanges ; l'éthanol et la N-méthyl-pyrrolidone étant particulièrement préférés.

Selon une forme particulière de réalisation de l'Invention et lorsque le DMSO est utilisé à titre de solvant, celui-ci est alors de préférence utilisé en quantités minimales ou en association minoritaire avec un des autres solvants listés ci-dessus.

Les compositions injectables gélifiantes conformes à l'Invention peuvent être préparées par dissolution de la quantité voulue de polymère(s) linéaire(s) dans le solvant miscible à l'eau, auquel on ajoute ensuite la quantité voulue de polymère(s) réticulé(s), de préférence préalablement préparé(s) sous forme de particules (microparticules). Les mélanges sont de préférence réalisés sous agitation magnétique afin notamment de faciliter la répartition homogène du ou des polymères réticulés au sein de la solution de polymère(s) linéaires.

Au cours de cette préparation, les solutions intermédiaires à base de copolymère linéaire comprenant au moins un copolymère linéaire de HPMA et de AN, et/ou au moins un copolymère de HPMA et de TBA, et/ou au moins un copolymère de HPMA et de AAMA et au moins un solvant biocompatible miscible à l'eau tel que par exemple décrit ci-dessus sont nouvelles en soi et constituent à ce titre un autre objet de l'Invention ; étant entendu que lorsque ladite solution intermédiaire renferme un copolymère linéaire d'hydroxypropylméthacrylate et d'acrylonitrile alors le solvant n'est pas le DMSO ni le diméthylformamide.

Les compositions injectables gélifiantes conformes à l'Invention peuvent en outre renfermer un ou plusieurs adjuvants choisis parmi les colorants (afin de rendre la composition visible lors de son injection) ; les marqueurs en imagerie tels que les agents contrastants pour l'imagerie aux rayons X comme par exemple les produits iodés et les poudres métalliques dont le tantale et le tungstène ou pour l'imagerie ultrasonores ou IRM, les produits radioactifs diagnostiques ou thérapeutiques ; les agents anti-inflammatoires ; les agents angiogéniques ; les anti-mitotiques ; les inhibiteurs de l'angiogénèse ; les facteurs de croissance ; les vitamines ; les hormones ; les protéines ; les vaccins ; les peptides ; les antiseptiques ; les antimicrobiens tels que les antibiotiques ; et de manière générale tout agent à visée thérapeutique, préventive ou diagnostique.

En fonction de leur solubilité vis-à-vis du solvant biocompatible utilisé, ces adjuvants peuvent être incorporés à la composition injectable gélifiante conforme à l'Invention sous forme de suspension, de solution ou bien encore être incorporés au sein des particules de polymère réticulé ou lié aux polymères (linéaires et/ou réticulés) par exemple par l'intermédiaire d'une liaison chimique.

Bien entendu, l'homme de l'art veillera à cette occasion à ce que le ou les adjuvants éventuellement utilisés soient compatibles avec les propriétés intrinsèques attachées à la composition injectable gélifiante conforme à l'Invention.

La présente Invention a également pour objet l'utilisation d'au moins une composition injectable gélifiante telle que décrite précédemment pour le comblement de conduits et cavités. En particulier, la composition injectable gélifiante conforme à l'Invention peut notamment être utilisée pour la réalisation d'occlusions vasculaires thérapeutiques (embolisations), pour la reconstruction tissulaire, le traitement du reflux gastro-oesophagien ou de l'incontinence urinaire, l'implantation percutanée, ou bien encore pour la réduction des rides.

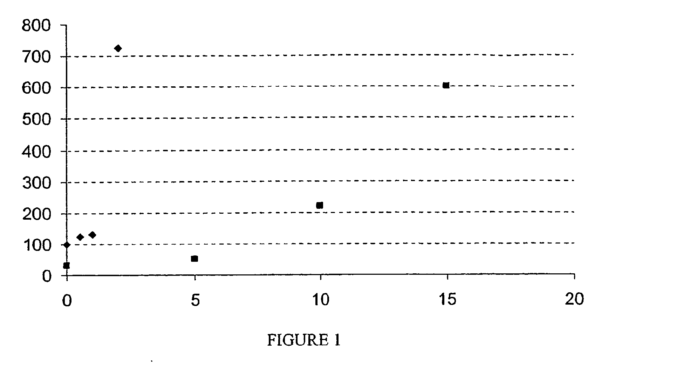

Outre les dispositions qui précèdent, l'Invention comprend encore d'autres dispositions qui ressortiront de la description qui va suivre, qui se réfère à un exemple de préparation de compositions injectables gélifiantes renfermant un polymère linéaire de p(HEMA) et des particules de polymère réticulé à différents taux de réticulation, à un exemple concernant une étude comparative de compositions injectables à base de polymère linéaire renfermant ou non des particules de polymère réticulé, à un exemple de réalisation d'une embolisation artérielle chez le mouton avec une composition injectable conforme à l'Invention comparativement à une composition ne faisant pas partie de l'Invention, ainsi qu'à la figure 1 annexée qui représente la viscosité de compositions injectables à base de HPMA-TBA ou de HPMA-AAMA à titre de polymère linéaire en fonction de la concentration en particules de polymère réticulé.

Il doit être bien entendu toutefois que ces exemples sont donnés uniquement à titre d'illustration de l'objet de l'Invention, dont ils ne constituent en aucune manière une limitation.

## EXEMPLE 1 : PRÉPARATION DE COMPOSITIONS GELIFIANTES INJECTABLES RENFERMANT UN POLYMERE p(HEMA) CHARGE PAR DIFFERENTES PARTICULES DE POLYMERE RETICULE

### 1) Préparation d'une solution de p(HEMA) linéaire

On prépare une solution de p(HEMA) (vendu sous la référence 18894-100 par la société Polysciences Inc. USA) à 12 % (m/V) dans de l'éthanol à 96,2 %.

### 2) Synthèse et préparation et étude du gonflement dans l'eau de particules de copolymères HEMA-Trisacryl®

On prépare des particules composées de copolymères acryliques (HEMA et/ou Trisacryl®) dans différentes proportions et à différents taux de réticulation. Pour la clarté de la description, seule la synthèse de particules composées de 50 % de HEMA et de 50 % de Trisacryl est présentée dans cet exemple. La synthèse de particules ayant d'autres pourcentages en chacun de ces deux monomères est réalisée selon le même procédé en faisant simplement varier les quantités de monomères utilisées. A titre d'exemple, pour la synthèse de particules composées à 100 % de HEMA, il suffit d'utiliser une quantité double de HEMA sans utiliser de Trisacryl®, et inversement pour des particules composées à 100 % de Trisacryl®.

Synthèse et préparation des particules

• Réactifs utilisés :

- HEMA : monomère 2-hydroxyéthyl méthacrylate 97 % vendu par la société Aldrich sous la référence 12,863-5 ; lot 05808 KI ;
- Trisacryl® : monomère de Trisacryl® en poudre vendu par la société Biosepra, France, sous la référence 5014 ;
- MBA : méthylène bis acrylamide (agent de réticulation) vendu par la société Biosepra sous la référence 7857 ;
- Agent amorceur : Ammonium peroxodisulphate vendu par la société Prolabo sous la référence 21300-293 ;
- TMEDA : tetraméthyléthylènediamine vendu par la société Acros Organics.

• Synthèse

La synthèse est effectuée par polymérisation radicalaire en solution.

Pour réaliser des particules composées de 50 % de HEMA et de 50 % de Trisacryl®, on verse dans un premier bécher 33 ml d'eau distillée, 5 g de monomère de Trisacryl®, 5 ml de monomère HEMA et 100 mg de MBA (soit 1 % d'agent réticulant m/m). Le mélange obtenu est chauffé à une température d'environ 50°C et agité afin de faciliter la dissolution des différents réactifs. La température et l'agitation sont maintenues durant toute la durée de la synthèse. Un bullage à l'argon permet d'éliminer les molécules d'$O_2$ qui pourraient gêner la réaction en captant des radicaux libres.

Dans un second bécher, on dissout 250 ml d'agent amorceur dans 1 ml d'eau désionisée. La solution est agitée magnétiquement puis versée dans le mélange contenu dans le premier bécher. Si la réaction ne démarre pas au bout de quelques minutes, on ajoute quelques gouttes de TMEDA qui favorise également l'amorçage de la synthèse.

Au bout de quelques minutes, la solution prend masse et l'agitation et le chauffage sont alors arrêtés. Le polymère réticulé obtenu, constitué de 50 % de HEMA et de 50 % de Trisacryl®, est découpé en petits fragments, puis mis à tremper dans un grand bécher d'eau désionisé. On procède ainsi à deux lavages successifs.

• Préparation des particules

Après lavage et élimination de l'eau, le polymère est déposé dans un cristallisoir et mis à sécher dans une étuve puis dans une étude à vide.

Après séchage, le polymère est réduit à l'état de poudre dans un mortier automatique pendant plusieurs heures. Il est ensuite tamisé sur tamis de 40 μm. Les particules de polymère réticulé sont alors stockées dans des flacons en verre, placés dans un dessiccateur contenant un produit dessiccant.

• Etude du gonflement des particules de polymères réticulés dans l'éthanol

Pour chacun des polymères réticulés préparés, les mesures de gonflement des particules ont été réalisées dans des éprouvettes graduées en utilisant 1 ml de particules sèches.

Après la mise en suspension des particules dans 50 ml d'éthanol, les particules sont laissées à sédimenter. Lorsque la sédimentation est stable, le volume des particules est mesuré. L'éthanol est alors remplacé par 50 ml d'eau. L'homogénéisation est effectuée par l'intermédiaire d'un Vortex® et en mélangeant à l'aide d'une pipette Pasteur. Ces opérations sont répétées plusieurs fois (au moins trois fois) afin d'éliminer tout l'éthanol. Lorsque les particules ont sédimenté, le volume des particules est à nouveau mesuré.

La variation du volume total ($\tau$) (en pourcentage) est définie par la formule suivante :

$$\tau = [(\text{Volume des particules dans l'eau } (V_{eau}) - \text{Volume des particules sèches } (V_{sec})) / V_{sec}] \times 100$$

La variation de volume (en pourcentage) des particules sèches dans l'éthanol ($\tau V1$) est calculée par la formule suivante :

$$\tau V1 = (V_{éthanol} - V_{sec})/V_{sec}) \times 100$$

La variation de volume (en pourcentage) des particules lors du passage de l'éthanol dans l'eau ($\tau V2$) est calculée par la formule suivante :

$$\tau V2 = (V_{eau} - V_{éthanol})/V_{éthanol}) \times 100$$

dans laquelle $V_{éthanol}$ est le volume des particules dans l'éthanol.

Les variations de volumes (en pourcentage) observées pour les différentes particules préparées sont reportées dans le Tableau I ci-après :

**TABLEAU 1**

| Composition des particules (%) | | | $\tau v1$ | $\tau v2$ | $\tau$ |
|---|---|---|---|---|---|
| HEMA | Trisacryl® | MBA | | | |
| 100 | 0 | 10 | 190 | -17 | 140 |
| 50 | 50 | 10 | 100 | 60 | 220 |
| 0 | 100 | 10 | 20 | 300 | 380 |
| 100 | 0 | 4 | 200 | -20 | 140 |
| 50 | 50 | 4 | 130 | 91 | 340 |
| 0 | 100 | 4 | 30 | 330 | 460 |

(suite)

| Composition des particules (%) | | | $\tau v1$ | $\tau v2$ | $\tau$ |
|---|---|---|---|---|---|
| HEMA | Trisacryl® | MBA | | | |
| 50 | 50 | 2 | 100 | 110 | 320 |
| 100 | 0 | 1 | 240 | 9 | 210 |
| 50 | 50 | 1 | 130 | 126 | 420 |
| 0 | 100 | 1 | 20 | 550 | 680 |

L'ensemble de ces résultats montre que les particules de polymère réticulé obtenues à partir d'une solution contenant 100 % de HEMA et 1 % d'agent de réticulation gonflent beaucoup (240 %) dans l'éthanol. Les particules de polymère réticulé obtenues à partir d'une solution contenant 50 % de HEMA et 50 % de Trisacryl ainsi que les particules constituées à 100 % de Trisacryl®, et présentant un taux de réticulation de 1 % gonflent beaucoup dans l'eau (respectivement 420 et 680 %).

**3) Préparation de compositions injectables à base de p(HEMA) et de particules de polymère réticulé (Compositions conformes à l'Invention)**

a) Etude de la viscosité de compositions de p(HEMA) linéaire dans l'éthanol chargées en particules de p(HEMA) réticulé et comparaison avec une solution ne contenant pas de particules

On prépare des compositions de p(HEMA) linéaire à 12 % (m/V) dans l'éthanol à 96,2° selon le protocole décrit ci-dessus en 1) contenant ou non 10 % (m/V) de particules constituées uniquement de p(HEMA) réticulé à 1, 4 ou 10 % (m/V) selon le protocole décrit ci-dessus en 2).

La viscosité des compositions a été mesurée à l'aide d'un rhéomètre RhéoStress ® RS 100, Hanke, piloté en contrainte et vendu par la société RHEO, Champlan, France. Une géométrie plateau/plateau a été utilisée. Le protocole consistait en une montée en contrainte de cisaillement de 0 à 500 Pa sur une durée de deux minutes à 20°C.

La viscosité de ces compositions a été comparée à celle de la solution préparée ci-dessus en 1) de p(HEMA) à 12 (m/V) dans l'éthanol et ne contenant pas de particules de polymère réticulé.

Les résultats obtenus sont reportés dans le tableau II ci-après :

**TABLEAU II**

| Compositions injectables | Viscosité en centipoises (cP) |
|---|---|
| p(HEMA) (12%) * | 100 |
| p(HEMA) (12%) + 10 % de particules p(HEMA) réticulé à 1 % | 2500 |
| p(HEMA) (12%) + 10 % de particules p(HEMA) réticulé à 4 % | 1000 |
| p(HEMA) (12%) + 10 % de particules p(HEMA) réticulé à 10 % | 500 |
| *: solution comparative ne faisant pas partie de l'Invention | |

Ces résultats montrent que la viscosité des compositions injectables conformes à l'Invention est beaucoup plus élevée que celle de la solution ne contenant pas de particules et que la viscosité des compositions conformes à l'Invention est d'autant plus élevée que le taux de réticulation du polymère formant les particules est faible.

b) Injection dans l'eau de compositions de p(HEMA) dans l'éthanol chargées en particules de Trisacryl réticulé ou en particules de p(HEMA) réticulé et comparaison avec une solution de p(HEMA) dans l'éthanol ne contenant pas de particules de polymère réticulé

On prépare différentes compositions de p(HEMA) linéaire à 12 % (m/V) dans l'éthanol à 96,2° selon le protocole décrit ci-dessus en 1) contenant 10 % (m/V) de particules constituées uniquement de Trisacryl® réticulé à 10 % (m/V) ou 10 % (m/V) de particules uniquement constituées de p(HEMA) réticulé à 10 % (m/V) selon le protocole décrit ci-dessus en 2).

Pour ce faire, les particules de Trisacryl® sont pesées directement dans un tube en verre ou versées à l'aide

7

d'un entonnoir. L'ajout de la solution de p(HEMA) linéaire dans l'éthanol se fait avec une pipette automatique. Les particules ont été préalablement réparties sur les cotés des tubes afin de faciliter l'homogénéisation au Vortex® de la suspension. Un barreau magnétique est introduit dans la suspension. La composition est ensuite laissée à homogénéiser par agitation magnétique pendant 12 heures.

A titre comparatif on étudie également le comportement lors de l'injection dans l'eau de la solution de p(HEMA) linéaire à 12 % (m/V) dans l'éthanol telle préparée ci-dessus en 1).

### 4) Injection dans l'eau

L'injection dans l'eau est réalisée dans les conditions suivantes : 1 ml de chacune des compositions ainsi préparées est injecté dans un bécher rempli d'eau désionisée à l'aide d'une seringue équipée d'une aiguille d'un diamètre de 0,6 mm. Un mouvement de rotation de la seringue dans le bécher permet de créer un léger cisaillement.

Les résultats obtenus sont les suivants :

- Composition de p(HEMA) linéaire à 12 % (m/V) dans l'éthanol contenant 10 % (m/V) de particules de p(HEMA) réticulé à 10 % (m/V) : les particules et le polymère ont une affinité l'un pour l'autre, ce qui entraîne une forte augmentation de la viscosité, mais la solidité du précipité de polymère n'est pas encore suffisante pour former une matrice de polymère qui piège les particules de p(HEMA) de façon assez solide pour piéger les particules de p (HEMA) et résister à un cisaillement important. Cette composition conforme à l'Invention est donc plus particulièrement destinée à être utilisée pour le comblement de conduits ou de cavités qui ne sont pas soumis à un flux trop important.

  A titre de comparaison :

  - L'injection de la solution de p(HEMA) dans l'éthanol sans particule de polymère réticulé conduit à l'apparition d'un précipité floconneux très faiblement cohésif. Cette expérience démontre que le p(HEMA) linéaire ne peut pas être utilisé seul en solution dans un solvant miscible à l'eau dans le but de former un précipité cohésif.
  - Composition de p(HEMA) linéaire à 12 % (m/V) dans l'éthanol contenant 10 % (m/V) de particules de Trisacryl® réticulé à 10 % (m/V) : lors de l'injection dans l'eau, les particules de p(HEMA) réticulé et le polymère p(HEMA) linéaire se séparent, le polymère linéaire formant des filaments peu cohésifs. Les particules et le polymère n'ayant aucune affinité l'un pour l'autre ne forment pas de gel cohésif.

### EXEMPLE 2 : PREPARATION DE COMPOSITIONS INJECTABLES CONTENANT DIFFERENTS COPOLYMERES A BASE D'HYDROXYPROPYLMETHACRYLATE (HPMA) ET DES PARTICULES DE COPOLYMERES TRISACRYL®/n(HEMA) (50/50)

### A) PREPARATION ET ETUDE DE SOLUTIONS COMPARATIVES COMPRENANT DES COPOLYMERES A BASE DE HPMA SANS PARTICULES DE POLYMERE RETICULE (solutions ne faisant pas partie de l'Invention)

### 1) Préparation de solutions à base de HPMA

a) Synthèse des copolymères

• Réactifs utilisés :

- HPMA : 2-hydroxypropyl méthacrylate vendu par la société Polysciences Inc. sous la référence 00730,
- AIBN : 2,2'-azo bis(2-méthylpropionitrile) à 98 % vendu par la société Acros Organics sous la référence 201-132-3,
- AN : acrylonitrile vendu par la société Aldrich sous la référence 11,021-3,
- TBA : N-*ter*-butylacrylamide à 97 %, vendu par la société Aldrich sous la référence 41,177-9,
- AAMA : acétoacétoxyéthylméthacrylate vendu par la société Acros Organics sous la référence 247950250,
- Ethanol absolu
- Ethanol à 96,2 %

• Synthèse

Les différents réactifs utilisés dans les synthèses et leurs proportions sont indiqués dans le tableau III ci-après :

**TABLEAU III**

| Copolymère | Monomère (ml) | Co-Monomère (ml) | AIBN (mg) | Ethanol (ml) |
|---|---|---|---|---|
| **HPMA-AN** | HPMA : 10 | AN : 3 | 50 | 30 |
| **HPMA-TBA** | HPMA : 10 | TBA : 2 | 50 | 30 |
| **HPMA-AAMA** | HPMA : 5 | AAMA : 1,5 | 25 | 15 |

On verse dans un ballon bicol de 1 litre, 10 ml de monomère 1 de HPMA. On ajoute ensuite 50 mg d'AIBN (agent amorceur de la copolymérisation), et 30 ml d'éthanol absolu et éventuellement la quantité indiquée ci-dessus de co-monomère 2 (AN, TBA ou AAMA).

Une colonne réfrigérante est placée sur le ballon, un bain-Marie permet de régler la température du mélange réactionnel. Un agitateur ovoïde permet d'homogénéiser la solution. On effectue un bullage à l'argon pendant 10 minutes afin d'éliminer les molécules d'O qui pourraient capter les radicaux libres et gêner la réaction de polymérisation. Lorsque la bullage est terminé, le bain-Marie est porté à une température comprise entre 60 et 70 °C. L'agitation est maintenue pendant 10 heures, puis on ajoute ensuite 20 ml d'éthanol afin de diminuer la viscosité du mélange. On procède ensuite à deux précipitations successives.

Première précipitation : on verse le mélange réactionnel dans un bécher de 5 litres, contenant 4 litres d'eau désionisée. Au contact de l'eau, le mélange réactionnel précipite et forme un voile de polymère. Ce voile est alors enroulé autour d'un agitateur en verre en forme d'ancre et sorti du bécher. Le polymère est ensuite redissous dans 500 ml d'éthanol absolu à une température de 70 °C afin de le laver des impuretés qui pourraient rester (monomères, agent amorceur). La solution de polymère est ensuite mise à refroidir.

Deuxième précipitation : la solution est ensuite précipitée une seconde fois comme décrit ci-dessus.

Le polymère précipité récupéré est alors séché à l'étuve à une température d'environ 50°C pendant plusieurs heures, puis à l'étuve sous vide pendant environ 12 heures. Lorsque le polymère est bien sec, il est cassé en petits morceaux manuellement et conditionné dans des flacons en plastiques hermétiques.

## 2) Mesures de la viscosité des différentes solutions de polymères

Les solutions de polymère sont préparées en dissolvant chacun des polymères préparés ci-dessus (HPMA-AN, HPMA-TBA et HPMA-AAMA) dans un solvant donné (éthanol absolu, N-méthyl-pyrrolidone (NMP) ou éthyl-lactate). Les concentrations sont établies en masse de polymère par volume de solvant (par exemple : 20 % = 20 g de polymère pour 100 ml de solvant).

La viscosité des différentes solutions de polymère à différentes concentrations dans les trois solvants a été mesurée sur un rhéomètre CSL[2] 100 vendu par la société TA Instruments, USA, à l'aide d'une géométrie cône/plateau. Chacune des solutions testées a été soumise à une montée en contrainte par palier de 0,5 à 5Pa, suivie d'une diminution de contrainte de 5 à 0,5 Pa. Les mesures ont été réalisées à une température de 20 °C.

Les viscosités obtenues pour chacune des solutions de polymère testées sont reportées dans le tableau IV ci-après :

**TABLEAU IV**

| Solvant | Concentration en polymère (%) | Viscosités mesurées (en cP) | | |
|---|---|---|---|---|
| | | **HPMA-AN** | **HPMA-TBA** | **HPMA-AAMA** |
| **Ethanol** | **5** | 5 | 8 | 8 |
| **Ethanol** | **10** | 22 | 30 | 27 |
| **Ethanol** | **15** | 72 | 75 | 84 |
| **Ethanol** | **20** | 202 | 164 | 260 |
| | | | | |
| **NMP** | **5** | 14 | 18 | 17 |
| **NMP** | **10** | 47 | 62 | 59 |
| **NMP** | **15** | 109 | 146 | 161 |
| **NMP** | **20** | 222 | 334 | 371 |

(suite)

| Solvant | Concentration en polymère (%) | Viscosités mesurées (en cP) | | |
|---|---|---|---|---|
| | | HPMA-AN | HPMA-TBA | HPMA-AAMA |
| | | | | |
| Ethyl-lactate | 5 | 16 | 19 | 25 |
| Ethyl-lactate | 10 | 49 | 65 | 83 |
| Ethyl-lactate | 15 | 140 | 180 | 251 |
| Ethyl-lactate | 20 | 350 | 428 | 767 |

Ces résultats montrent qu'en terme de viscosité, l'éthanol absolu est le moins bon solvant pour les trois polymères étudiés puisqu'il induit les viscosités les plus faibles. Cependant, l'éthanol absolu présente, parmi les trois solvants utilisés dans cet exemple, la plus forte diffusivité dans l'eau, avantage décisif pour la séparation de phases d'une solution d'embolisation.

Les paramètres de solubilité des solvants étudiés, tels que donnés par exemple dans l'ouvrage "Polymer Science Dictionnary, Essex (England), Elsevier Science Publishers, 1989 est de 26 $(MPa)^{1/2}$ pour l'éthanol, de 20 $(MPa)^{1/2}$ pour l'éthyl-lactate et de 23 $(MPa)^{1/2}$ pour le NMP. L'eau ayant un paramètre de 47 $(MPa)^{1/2}$, c'est l'éthanol qui a la plus forte diffusivité dans l'eau.

### 3) Injection dans l'eau

L'injection dans l'eau des solutions de ces trois copolymères linéaires (HPMA-AN, HPMA-TBA et HPMA-AAMA) à 20 % (m/V) dans l'éthanol conduit à des précipités plus cohésifs que celui de la solution de p(HEMA) ne contenant pas de particules de polymère réticulé préparée et testées ci-dessus à l'exemple 1, mais de faible volume comparé au volume injecté, en raison du retrait important du solvant qui se produit lors de la mise en contact avec l'eau.

Par conséquent, ces résultats montrent que ces trois copolymères linéaires ne peuvent pas être utilisés seuls en solution dans un solvant miscible à l'eau dans le but de former un précipité cohésif.

### B) PREPARATION ET ETUDES DE COMPOSITIONS COMPRENANT DES COPOLYMERES A BASE DE HPMA ET DES PARTICULES DE TRISACRYL®-HEMA (compositions injectables conformes à l'Invention)

### 1) Préparation des compositions injectables

On prépare différentes compositions injectables conformes à l'Invention renfermant 10 % (m/V) d'un copolymère de HPMA-TBA ou de HPMA-AAMA, tels que préparés ci-dessus en A) 1) et renfermant :

-   0,1 ; 1 ou 2 % (m/V) de particules de copolymère statistique 50 % Trisacryl® - 50 % HEMA, réticulées à 2 % (m/V) telles que préparées ci-dessus à l'exemple 1, paragraphe 2) pour les compositions à base de copolymère HPMA-AAMA et
-   5 ; 10 ou 15 % de particules de copolymère statistique 50 % Trisacryl® - 50 % HEMA, réticulées à 2 % (m/V) telles que préparées ci-dessus à l'exemple 1, paragraphe 2) pour les compositions à base de copolymère HPMA-TBA.

Deux solutions comparatives sans particules de copolymères réticulé mais renfermant respectivement 10 % (m/V) d'un copolymère de HPMA-TBA ou 10 % (m/V) de HPMA-AAPA ont également été préparées.

La viscosité des compositions et solutions comparatives ainsi préparées a été mesurée selon la méthode décrite ci-dessus dans cet exemple, paragraphe 2), mais avec un rhéomètre CSL 100 vendu par la société TA Instruments, USA, à géométrie plateau / plateau.

Les viscosités obtenues en fonction de la quantité de particules contenues dans chacune des compositions sont représentées sur la figure 1 annexée sur laquelle la viscosité (en mPa.s) est exprimée en fonction de la concentration en particules (m/V), les carrés noirs représentant la viscosité des compositions à base de copolymère HPMA-TBA et les losanges noirs représentant la viscosité des compositions à base de copolymère HPMA-AAMA.

Ces résultats montrent que la viscosité des compositions à base de copolymère de HPMA-AAMA augmente beaucoup plus vite en fonction de la concentration en particules que celle des compositions à base de copolymère HPMA-TBA. Ces résultats indiquent donc que les interactions entre le copolymère HPMA-AAMA et les particules sont plus fortes que les interactions entre le copolymère HPMA-TBA et les particules.

**2) Injection dans l'eau des compositions et solutions de copolymères à base de HPMA et renfermant ou non des particules de Trisacryl®-HEMA**

On prépare des compositions injectables renfermant ou non (solutions comparatives) des particules de copolymère statistique 50 % Trisacryl® - 50 % HEMA, réticulées à 2 % (m/V) telles que préparées ci-dessus à l'exemple 1, paragraphe 2). La composition et la viscosité des différentes compositions et solutions comparatives injectables préparées sont détaillées dans le tableau V ci-dessous :

**TABLEAUV**

| Compositions et Solutions injectables | Nature du copolymère | Quantité de copolymère (% m/V) | Nature du solvant | Quantité de particules (% m/V) | Viscosité (mPa.s) |
|---|---|---|---|---|---|
| 1 * | HPMA-AN | 20 | Ethanol | - | 200 |
| 2 * | HPMA-AAMA | 20 | Ethanol | - | Nd |
| 3 * | HPMA-AAMA | 20 | Ethanol | - | Nd |
| 4 * | HPMA-AAMA | 20 | Ethanol | - | Nd |
| 5 * | HPMA-AAMA | 20 | Ethanol | - | Nd |
| 6 | HPMA-AAMA | 10 | Ethanol | 2 | 700 |
| 7 | HPMA-AAMA | 10 | Ethanol | 1 | 700 |
| 8 | HPMA-AAMA | 10 | NMP | 1 | Nd |
| 9 | HPMA-AAMA | 10 | NMP | 3 | Nd |
| 10 * | HPMA-TBA | 20 | Ethanol | - | 160 |
| 11 | HPMA-TBA | 20 | Ethanol | 10 | Nd |
| * : Solutions comparatives ne faisant pas partie de l'Invention | | | | | |

L'injection dans l'eau de ces différentes compositions et solutions comparatives a été réalisée sur un banc dynamique afin de simuler une embolisation. Le banc dynamique est constitué d'un réservoir rempli d'eau et situé en hauteur par rapport au lieu d'écoulement à pression constante de l'eau dans une tubulure de 2 mm de diamètre. L'injection de chacune des compositions ou solutions 1 à 11 à tester est faite avec une seringue de 5 ml dans la tubulure au travers d'une aiguille de 0,3 mm de diamètre intérieur et de 4,1 mm de diamètre externe qui traverse la paroi de la tubulure et dont l'extrémité est placée au centre de la tubulure. Les dimensions du banc (longueur de l'aiguille, distance entre l'extrémité de l'aiguille et l'arrivée d'eau, etc...) sont choisies de façon à obtenir un écoulement laminaire dans la tubulure. La vitesse moyenne de l'eau dans la tubulure est de $60 \pm 5$ cm/seconde. Un bécher et une balance permettent de mesurer le débit dans la tubulure. Le débit est réglé en diminuant la lumière de l'arrivée d'eau dans la tubulure en amont de l'aiguille d'injection, à l'aide d'une ou de deux pinces de Mohr. Une caméra vidéo connectée à un ordinateur portable permet de filmer les injections afin de comparer et d'évaluer les différents gels formés lors de l'injection des compositions et solutions dans l'eau. Une échelle graduée en centimètres, positionnée en regard de la tubulure et dont le zéro est aligné sur l'extrémité de l'aiguille d'injection située au centre de la tubulure, permet de mesurer la longueur de l'embole formée après injection.

Les résultats obtenus pour chacune des compositions ou solutions comparatives injectables 1 à 11 ont été formulés sous la forme d'un score composite qui permet de caractériser le pouvoir d'embolisation de chacune des compositions et de le comparer entre elles. Ce score est composé des trois éléments suivants :

- la cohésion de la composition injectée lors de sa solidification au contact de l'eau : elle est appréciée soit en fonction de la fragmentation ou non de la composition injectée, soit sur sa capacité à résister au flux ;
- le taux d'occlusion par rapport au diamètre du tube : il correspond au pourcentage de la lumière du tube occlus par la composition ou solution injectée ;
- la longueur de l'embole : elle quantifie la rapidité d'occlusion de la lumière du banc, plus l'embole permettant l'occlusion est court, plus la composition d'embolisation est efficace.

En ce qui concerne le critère de cohésion de la composition ou solution injectée, la notation est effectuée de la manière suivante :

- le gel se fragmente dès la sortie de l'aiguille : +
- le gel d'étire dans le flux, puis se fragmente : + +
- le gel s'étire dans le flux sans se fragmenter : + + +
- le gel est quasi immobile, il avance peu dans le flux : + + ++

En ce qui concerne le taux d'occlusion, la notation est effectuée de la manière suivante :

- occlusion inférieure à 33 % : +
- occlusion comprise entre 33 et 66 % : + +
- occlusion comprise entre 66 % et 99 % : + + +
- occlusion complète : + + + +
- occlusion complète avec reflux vers l'aiguille : + + + + +

En ce qui concerne la longueur de l'embole, et en cas d'occlusion, la notation est effectuée de la manière suivante :

- longueur de l'embole supérieure à 3 cm : +
- longueur de l'embole comprise entre 3 et 2 cm : + +
- longueur de l'embole inférieure à 1 cm : + + +

Le total du nombre de croix obtenues pour chacun des critères étudié conduit une note globale qui est le score composite dont le maximum possible est 12.

Les résultats obtenus pour chacune des compositions ou solutions comparatives injectables 1 à 11 sont reportés dans le tableau VI ci-après :

### TABLEAU VI

| Compositions ou Solutions injectables | Cohésion | Taux d'occlusion | Longueur de l'embole | Score composite |
|---|---|---|---|---|
| 1 * | + | + | Non formée | 2 |
| 2 * | + + + + | + + + + | + | 9 |
| 3 * | + | + | Non formée | 2 |
| 4 * | + + + + | + + | Non formée | 6 |
| 5 * | + + | + + | Non formée | 4 |
| 6 | + + | + | Non formée | 3 |
| 7 | + + | + + | Non formée | 4 |
| 8 | + + | + | Non formée | 3 |
| 9 | + + | + | Non formée | 3 |
| 10 * | + + + + | + + + + | + | 9 |
| 11 | ++++ | +++++ | ++ | 11 |
| * : Solutions comparatives ne faisant pas partie de l'Invention | | | | |

Ces résultats montrent que la solution comparative injectable n° 10 à base de copolymère HPMA-TBA sans particules de polymère réticulé conduit à de meilleurs résultats en terme d'embolisation que les solutions injectables à base des copolymères HPMA-AN ou HPMA-AAMA.

De plus, l'ajout de particules de polymères statistiques 50 % Trisacryl® - 50 % HEMA à 2 % de réticulation (m/V) à cette solution à base de copolymère NPMA-TBA à 20 % dans l'éthanol (composition n°11) améliore le pouvoir d'embolisation de la composition n°10 ne contenant pas lesdites particules.

**3) Etude du gonflement dans l'eau d'une composition injectable conforme à l'Invention**

On prélève dans une seringue 1 ml d'une composition injectable de HPMA-TBA à 10 % (m/v) dans l'éthanol renfermant 15 % (m/v) de particules de Trisacryl®/HEMA (50/50) réticulé à 2 % (m/V).

Cette composition est injectée dans un bécher rempli d'eau sous agitation magnétique de manière à former un embole stable et suffisamment compact pour être manipulé. Lorsque l'embole est formé, on le laisse reposer dans le bécher sous agitation magnétique faible. Après une quinzaine d'heure, l'aspect blanc et ferme de l'embole laisse supposer que les échanges de solvant dans l'échantillon sont terminés. L'embole est alors prélevé et placé dans une éprouvette graduée contenant un volume précis d'eau. La différence de volume observé après introduction de l'embole dans l'éprouvette correspond au volume de l'embole.

Dans cet exemple, le volume de l'embole est de 1,6 ml.

Ce résultat montre qu'après injection dans l'eau et solidification de la matrice de polymère linéaire, les particules gonflent et induisent un gonflement du gel de plus de 60 %.

**EXEMPLE 3 : REALISATION D'EMBOLISATIONS ARTERIELLES CHEZ LE MOUTON AVEC UNE COMPOSITION INJECTABLE CONFORME A L'INVENTION ET COMPARAISON AVEC UNE SOLUTION NE CONTENANT PAS DE PARTICULES DE POLYMERE RETICULE**

Le but de cet exemple est de comparer les effets obtenus en terme d'embolisation artérielle chez le mouton avec des compositions injectables conformes à l'Invention c'est-à-dire comprenant des particules de polymère réticulé et des solutions injectables non conformes à l'Invention, c'est-à-dire ne comprenant pas de particules de polymère réticulé.

Pour ce faire la solution injectable n°10 et la composition injectable n°11 telles que décrites ci-dessus dans le tableau V de l'exemple 2 ont été utilisées.

Avant utilisation, la solution et la composition sont stérilisées à une température de 70°C.

Chacune d'entre elles est ensuite rendue radio opaque par l'ajout et le mélange d'une poudre micronisée de tantale à la dose de 0,5 g par ml de composition.

L'injection de ces préparations a été réalisée chez le mouton, sous anesthésie générale : après abord fémoral avec un introducteur 5F, plusieurs artères intercostales ont été cathétérisées de façon sélective. Dans chacune d'entre elles, une injection de 0,5 ml de composition gélifiante est effectuée sous fluoroscopie. La répartition du gel est suivie sous fluoroscope pendant plusieurs minutes, puis contrôlée à intervalles réguliers pendant une heure.

La solution non conforme à l'Invention, c'est-à-dire ne renfermant pas de particules de polymère réticulé avance dans l'arbre vasculaire, poussée par le flux artériel, sur plusieurs centimètres pendant les premières minutes suivant l'injection. L'occlusion finalement obtenue est incomplète, distale et instable.

Par contre, la composition conforme à l'Invention, c'est-à-dire renfermant des particules de polymère réticulé en suspension avance peu sous le flux et reste localisée sous forme de gel à l'extrémité du cathéter. L'occlusion obtenue est complète, proximale et stable. Le contrôle radiographique effectué une heure après l'injection montre que la solution ne contenant pas les particules de polymère réticulé conduit à la formation de fragment de gel sur tout le parcours de l'artère alors que l'injection de la composition conforme à l'Invention conduit à la formation d'un embole proximal qui reste stable et en place.

**Revendications**

1.   Composition injectable gélifiante à base de polymères pour l'implantation intra-tissulaire et/ou intra-vasculaire, **caractérisée par le fait qu'**elle comprend :

     - au moins un polymère linéaire insoluble dans l'eau et soluble dans au moins un solvant miscible à l'eau,
     - au moins un polymère réticulé hydrophile et insoluble dans l'eau,
     - au moins un solvant biocompatible miscible à l'eau ; et **par le fait qu'**elle se présente sous la forme d'une suspension de particules dudit polymère réticulé hydrophile dans une solution dudit polymère linéaire.

2.   Composition injectable gélifiante selon la revendication 1, **caractérisée par le fait que** le ou les polymères linéaires sont choisis parmi les poly(acrylates d'alkyle), les poly(méthacrylates d'alkyle), les poly(cyanoacrylates d'alkyle), les poly(acétate de vinyle), les poly(butyrate de vinyle), les poly(vinyle formal), les poly(vinyle acétal), les poly(vinyle butyral), les poly(oxypropylène), les poly(oxytétraméthylène), les esters de cellulose insolubles dans l'eau, les esters de chitosane ou autres polysaccharides insolubles dans l'eau, les polylactides, les polyglycolides, les polycaprolactone, les esters de poly(acide malique), les esters de poly(acide maléique), les esters de poly(acide fumarique), et les copolymères ou dérivés linéaires insolubles dans l'eau comprenant ces composés.

3. Composition injectable gélifiante selon la revendication 2, **caractérisée par le fait que** le ou les polymères linéaires sont choisis parmi le polyhydroxyéthylméthacrylate, le poly(méthacrylate de méthyle), le polyhydroxypropylméthacrylate, les copolymères d'hydroxyéthylméthacrylate ou d'hydroxypropylméthacrylate et d'acrylonitrile, les copolymères d'hydroxyéthylméthacrylate ou d'hydroxypropylméthacrylate et de N-*ter*-butylacrylamide, les copolymères d'hydroxyéthylméthacrylate ou d'hydroxypropylméthacrylate et d'acétoacétoxyéthylméthacrylate, le poly-(N-acryloyl-2-amino-2-hydroxyméthyl-1,3-propanediol), le poly-(n-2-hydroxypropyl méthacrylamide), et leurs dérivés.

4. Composition injectable gélifiante selon la revendication 3, **caractérisée par le fait que** le ou les polymères linéaires sont choisis parmi les copolymères d'hydroxypropylméthacrylate et d'acrylonitrile, les copolymères d'hydroxypropylméthacrylate et de N-*ter*-butylacrylamide et les copolymères d'hydroxypropylméthacrylate et d'acétoacétoxyéthylméthacrylate.

5. Composition injectable gélifiante selon l'une quelconque des revendications précédentes, **caractérisée par le fait que** le ou les polymères linéaires représentent de 3 à 25 % (m/V).

6. Composition injectable gélifiante selon l'une quelconque des revendications précédentes, **caractérisée par le fait que** le ou les polymères réticulés hydrophiles sont choisis parmi les polymères issus de la réticulation des polymères linéaires insolubles dans l'eau tels que définis à la revendication 2.

7. Composition injectable gélifiante selon l'une quelconque des revendications 1 à 5, **caractérisée par le fait que** le ou les polymères réticulés hydrophiles sont choisis parmi les polymères issus de la réticulation de polymères linéaires solubles dans l'eau.

8. Composition injectable gélifiante selon la revendication 7, **caractérisée par le fait que** les polymères linaires solubles dans l'eau sont choisis parmi les alginates ; les dérivés de l'amidon ; les éthers de cellulose ; les acétates de cellulose de degré de substitution compris entre 0,6 et 0,8 ; les sulfates de cellulose ; les polysaccharides hydrosolubles ; les sels de chitosanes ; les polymères acryliques et méthacryliques ; les polyacrylamides et polyméthacrylamides substitués ou non ; les dérivés hydrolysés des poly(vinylacétates) ; les polymères dérivés du poly(oxyéthylène), la poly(éthylèneimine) ; les sels solubles de la poly(vinylpyridine) ; la poly(vinylpyrrolidone) ; les polyuréthanes ; leurs sels et leurs copolymères.

9. Composition injectable gélifiante selon la revendication 6, **caractérisée par le fait que** le ou les polymères réticulés sont choisis parmi les polymères réticulés d'hydroxyéthylméthacrylate, d'hydroxypropylméthacrylate ou de poly-(N-acryloyl-2-amino-2-hydroxyméthyl-1,3-propanediol), ainsi que parmi les copolymères réticulés d'hydroxyéthylméthacrylate et de poly-(N-acryloyl-2-amino-2-hydroxyméthyl-1,3-propanediol) ou d'hydroxypropylméthacrylate et de poly-(N-acryloyl-2-amino-2-hydroxyméthyl-1,3-propanediol).

10. Composition injectable gélifiante selon l'une quelconque des revendications précédentes, **caractérisée par le fait que** le taux de réticulation du polymère réticulé est compris entre 0,5 et 12 % (m/V).

11. Composition injectable gélifiante selon l'une quelconque des revendications précédentes, **caractérisée par le fait que** le ou les polymères réticulés représentent de 1 à 30 % (m/V).

12. Composition injectable gélifiante selon l'une quelconque des revendications précédentes, **caractérisée par le fait que** la taille des particules de polymère(s) réticulé(s) est comprise entre 1 et 1000 $\mu$m.

13. Composition injectable gélifiante selon l'une quelconque des revendications précédentes, **caractérisée par le fait qu'**elle comprend au moins un polymère linéaire d'hydroxyéthylméthacrylate ou d'hydroxypropylméthacrylate ou un copolymère linéaire à base d'hydroxyéthylméthacrylate ou d'hydroxypropylméthacrylate et des particules de polymères réticulés d'hydroxyéthylméthacrylate, d'hydroxypropylméthacrylate ou de poly-(N-acryloyl-2-amino-2-hydroxyméthyl-1,3-propanediol), et/ou de copolymères réticulés d'hydroxyéthylméthacrylate et de poly-(N-acryloyl-2-amino-2-hydroxyméthyl-1,3-propanediol) ou d'hydroxypropylméthacrylate et de poly-(N-acryloyl-2-amino-2-hydroxyméthyl-1,3-propanediol).

14. Composition injectable gélifiante selon la revendication 13, **caractérisée par le fait qu'**elle comprend :

- au moins un copolymère linéaire à base d'hydroxyéthylméthacrylate ou d'hydroxypropylméthacrylate, et
- au moins des particules de copolymères d'hydroxyéthylméthacrylate ou d'hydroxypropylméthacrylate et de poly-(N-acryloyl-2-amino-2-hydroxyméthyl-1,3-propanediol) réticulés.

15. Composition injectable gélifiante selon l'une quelconque des revendications précédentes, **caractérisée par le fait que** le ou les solvants biocompatibles miscibles à l'eau sont choisis parmi la N-méthyl-pyrrolidone, le diméthyléthylamide, le diméthyléther de diéthylèneglycol, l'éthyl-lactate, l'éthanol, le diméthoxyéthane, le diméthylsulfoxyde, le glycofurol et leurs mélanges.

16. Composition injectable gélifiante selon la revendication 15, **caractérisée par le fait que** lesdits solvants sont choisis parmi l'éthanol et la N-méthyl-pyrrolidone.

17. Composition injectable gélifiante selon l'une quelconque des revendications précédentes, **caractérisée par le fait qu'**elle renferme en outre un ou plusieurs adjuvants choisis parmi les colorants ; les marqueurs en imagerie ; les agents anti-inflammatoires ; les agents angiogéniques ; les anti-mitotiques ; les inhibiteurs de l'angiogénèse ; les facteurs de croissance ; les vitamines ; les hormones ; les protéines ; les vaccins ; les peptides ; les antiseptiques et les antimicrobiens.

18. Composition injectable gélifiante telle que définie à l'une quelconque des revendications 1 à 17, pour son utilisation dans le comblement de conduits et cavités.

19. Composition injectable gélifiante telle que définie à l'une quelconque des revendications 1 à 17, pour son utilisation pour la réalisation d'occlusions vasculaires thérapeutiques, pour la reconstruction tissulaire, le traitement du reflux gastro-oesophagien ou de l'incontinence urinaire, pour l'implantation percutanée ou pour la réduction des rides.

20. Solution intermédiaire à base de copolymère linéaire **caractérisée par le fait qu'**elle comprend :

- au moins un copolymère linéaire d'hydroxypropylméthacrylate et d'acrylonitrile, et/ou au moins un copolymère d'hydroxypropylméthacrylate et de N-*ter*-butylacrylamide et/ou au moins un copolymère d'hydroxypropylméthacrylate et d'acétoacétoxyéthylméthacrylate, et
- au moins un solvant biocompatible miscible à l'eau ; étant entendu que lorsque ladite solution intermédiaire renferme un copolymère linéaire d'hydroxypropylméthacrylate et d'acrylonitrile alors le solvant n'est pas le diméthylsulfoxyde ni le diméthylformamide.

## Claims

1. Injectable gel-forming composition based on polymers for intra-tissue and/or intravascular implantation, **characterised in that** it comprises:

- at least one straight-chain polymer which is insoluble in water and soluble in at least one water-miscible solvent,
- at least one hydrophilic cross-linked polymer which is insoluble in water,
- at least one water-miscible biocompatible solvent;

and **in that** it takes the form of a suspension of particles of said hydrophilic cross-linked polymer in a solution of said straight-chain polymer.

2. Injectable gel-forming composition according to claim 1, **characterised in that** the straight-chain polymer or polymers are selected from among polyalkylacrylates, polyalkylmethacrylates, polyalkylcyanoacrylates, polyvinylacetates, polyvinylbutyrates, polyvinyl formals, polyvinyl acetals, polyvinyl butyrals, poly(oxypropylenes), poly(oxytetramethylenes), water-insoluble cellulose esters, esters of chitosan or other water-insoluble polysaccharides, polylactides, polyglycolides, polycaprolactones, poly(malic acid) esters, poly(maleic acid) esters, poly(fumaric acid) esters and water-insoluble straight-chain copolymers or derivatives containing these compounds.

3. Injectable gel-forming composition according to claim 2, **characterised in that** the straight-chain polymer or polymers are selected from among polyhydroxyethyl methacrylate, poly(methyl methacrylate), polyhydroxypropyl methacrylate, copolymers of hydroxyethylmethacrylate or hydroxypropylmethacrylate and acrylonitrile, copolymers of hydroxyethylmethacrylate or hydroxypropylmethacrylate and N-tert. butylacrylamide, copolymers of hydroxyethyl methacrylate or hydroxypropylmethacrylate and acetoacetoxyethyl methacrylate, poly-(N-acryloyl-2-amino-2-hydroxymethyl-1,3-propanediol), poly-(n-2-hydroxypropylmethacrylamide) and the derivatives thereof.

4. Injectable gel-forming composition according to claim 3, **characterised in that** the straight-chain polymer or polymers are selected from among the copolymers of hydroxypropyl methacrylate and acrylonitrile, the copolymers of hydroxypropyl methacrylate and N-tert-butylacrylamide and the copolymers of hydroxypropyl methacrylate and acetoacetoxyethyl methacrylate.

5. Injectable gel-forming composition according to any one of the preceding claims, **characterised in that** the straight-chain polymer or polymers represent from 3 to 25% (m/V).

6. Injectable gel-forming composition according to any one of the preceding claims, **characterised in that** the hydrophilic cross-linked polymer or polymers are selected from among the polymers obtained by cross-linking the water-insoluble straight-chain polymers as defined in claim 2.

7. Injectable gel-forming composition according to any one of claims 1 to 5, **characterised in that** the hydrophilic cross-linked polymer or polymers are selected from among the polymers obtained by cross-linking water-soluble straight-chain polymers.

8. Injectable gel-forming composition according to claim 7, **characterised in that** the water-soluble straight-chain polymers are selected from among the alginates; starch derivatives; cellulose ethers; cellulose acetates with a degree of substitution of between 0.6 and 0.8; cellulose sulphates; water-soluble polysaccharides; chitosan salts; acrylic and methacrylic polymers; substituted or unsubstituted polyacrylamides and polymethacrylamides; hydrolysed poly(vinylacetate) derivatives; polymers derived from poly(oxyethylene), poly(ethyleneimine); the soluble salts of poly(vinylpyridine); poly(vinylpyrrolidone); the polyurethanes; the salts thereof and the copolymers thereof.

9. Injectable gel-forming composition according to claim 6, **characterised in that** the cross-linked polymer or polymers are selected from among the cross-linked polymers of hydroxyethyl methacrylate, hydroxypropyl methacrylate or poly-(N-acryloyl-2-amino-2-hydroxymethyl-1,3-propanediol) and from among the cross-linked polymers of hydroxyethyl methacrylate and poly-(N-acryloyl-2-amino-2-hydroxymethyl-1,3-propanediol) or hydroxypropyl methacrylate and poly-(N-acryloyl-2-amino-2-hydroxymethyl-1,3-propanediol).

10. Injectable gel-forming composition according to any one of the preceding claims, **characterised in that** the degree of cross-linking of the cross-linked polymer is between 0.5 and 12% (m/V).

11. Injectable gel-forming composition according to any one of the preceding claims, **characterised in that** the cross-linked polymer or polymers represent from 1 to 30% (m/V).

12. Injectable gel-forming composition according to any one of the preceding claims, **characterised in that** the particle size of the cross-linked polymer(s) is between 1 and 1000 $\mu$m.

13. Injectable gel-forming composition according to any one of the preceding claims, **characterised in that** it comprises at least one straight-chain polymer of hydroxyethyl methacrylate or hydroxypropyl methacrylate or a straight-chain copolymer based on hydroxyethyl methacrylate or hydroxypropyl methacrylate and particles of cross-linked polymers of hydroxyethyl methacrylate, hydroxypropyl methacrylate or poly-(N-acryloyl-2-amino-2-hydroxymethyl-1,3-propanediol), and/or cross-linked copolymers of hydroxyethyl methacrylate and poly-(N-acryloyl-2-amino-2-hydroxymethyl-1,3-propanediol) or hydroxypropyl methacrylate and poly-(N-acryloyl-2-amino-2-hydroxymethyl-1,3-propanediol).

14. Injectable gel-forming composition according to claim 13, **characterised in that** it comprises:

   - at least one straight-chain copolymer based on hydroxyethyl methacrylate or hydroxypropyl methacrylate, and
   - at least particles of cross-linked copolymers of hydroxyethyl methacrylate or hydroxypropyl methacrylate and poly-(N-acryloyl-2-amino-2-hydroxymethyl-1,3-propanediol).

15. Injectable gel-forming composition according to any one of the preceding claims, **characterised in that** the water-miscible biocompatible solvent or solvents are selected from among N-methyl-pyrrolidone, dimethylethylamide, diethyleneglycol dimethylether, ethyl lactate, ethanol, dimethoxyethane, dimethylsulphoxide, glycofurol and mixtures thereof.

16. Injectable gel-forming composition according to claim 15, **characterised in that** the said solvents are selected from among ethanol and N-methylpyrrolidone.

17. Injectable gel-forming composition according to any one of the preceding claims, **characterised in that** it further comprises one or more adjuvants selected from among the dyes; imaging markers; anti-inflammatory agents; angiogenic agents; anti-mitotics; angiogenesis inhibitors; growth factors; vitamins; hormones; proteins; vaccines; peptides; antiseptics and antimicrobials.

18. Injectable gel-forming composition as defined in any one of claims 1 to 17, for use in filling ducts and cavities.

19. Injectable gel-forming composition as defined in any one of claims 1 to 17, for use in producing therapeutic vascular occlusions, for tissue reconstruction, treating gastro-oesophageal reflux or urinary incontinence, for percutaneous implantation or for wrinkle reduction.

20. Intermediate solution based on straight-chain copolymer, **characterised in that** it comprises:

- at least one straight-chain copolymer of hydroxypropyl methacrylate and acrylonitrile, and/or at least one copolymer of hydroxypropyl methacrylate and N-tert. butylacrylamide and/or at least one copolymer of hydroxypropylmethacrylate and acetoacetoxyethyl methacrylate, and
- at least one water-miscible biocompatible solvent; it being understood that when said intermediate solution contains a straight-chain copolymer of hydroxypropyl methacrylate and acrylonitrile the solvent is not dimethylsulphoxide or dimethylformamide.

**Patentansprüche**

1. Injizierbare gelierende Zusammensetzung auf der Basis von Polymeren zur Implantation in Gewebe und/oder Gefäße, **dadurch gekennzeichnet, dass** sie umfasst:

- wenigstens ein lineares Polymer, das in Wasser unlöslich und in wenigstens einem mit Wasser mischbaren Lösungsmittel löslich ist,
- wenigstens ein hydrophiles und in Wasser unlösliches, vernetztes Polymer,
- wenigstens ein mit Wasser mischbares, biokompatibles Lösungsmittel;

und **dadurch**, dass sie in Form einer Suspension von Partikeln des hydrophilen, vernetzten Polymers in einer Lösung des linearen Polymers vorliegt.

2. Injizierbare gelierende Zusammensetzung gemäß Anspruch 1, **dadurch gekennzeichnet, dass** das lineare Polymer oder die linearen Polymeren ausgewählt ist/sind aus Polyalkylacrylaten, Polyalkylmethacrylaten, Polycyanoalkylacrylaten, Polyvinylacetat, Polyvinylbutyrat, Polyvinylformal, Polyvinylacetal, Polyvinylbutyral, Polyoxypropylen, Polyoxytetramethylen, in Wasser unlöslichen Celluloseestern, Estern von Chitosan oder anderen Polysacchariden, die in Wasser unlöslich sind, Polylactiden, Polyglycoliden, Polycaprolacton, Estern von Polyäpfelsäure, Estern von Polymaleinsäure, Estern von Polyfumarsäure und linearen, in Wasser unlöslichen Copolymeren oder Derivaten, die diese Verbindungen umfassen.

3. Injizierbare gelierende Zusammensetzung gemäß Anspruch 2, **dadurch gekennzeichnet, dass** das lineare Polymer oder die linearen Polymere ausgewählt ist/sind aus Polyhydroxyethylmethacrylat, Polymethylmethacrylat, Polyhydroxypropylmethacrylat, Copolymeren von Hydroxyethylmethacrylat oder Hydroxypropylmethacrylat und Acrylnitril, Copolymeren von Hydroxyethylmethacrylat oder Hydroxypropylmethacrylat und N-tert-Butylacrylamid, Copolymeren von Hydroxyethylmethacrylat oder Hydroxypropylmethacrylat und Acetoacetoxyethylmethacrylat, Poly(N-acryloyl-2-amino-2-hydroxymethyl-1,3-propandiol), Poly(n-2-hydroxypropylmethacrylamid) und ihren Derivaten.

4. Injizierbare gelierende Zusammensetzung gemäß Anspruch 3, **dadurch gekennzeichnet, dass** das lineare Polymer oder die linearen Polymere ausgewählt ist/sind aus Copolymeren von Hydroxypropylmethacrylat und Acrylnitril, Copolymeren von Hydroxypropylmethacrylat und N-tert-Butylacrylamid und Copolymeren von Hydroxypropylmethacrylat und Acetoacetoxyethylmethacrylat.

5. Injizierbare gelierende Zusammensetzung gemäß einem der vorangehenden Ansprüche, **dadurch gekennzeichnet, dass** das lineare Polymer oder die linearen Polymere 3 bis 25% (M/V) ausmachen.

6. Injizierbare gelierende Zusammensetzung gemäß einem der vorangehenden Ansprüche, **dadurch gekennzeichnet, dass** das hydrophile, vernetzte Polymer oder die hydrophilen, vernetzten Polymere ausgewählt ist/sind aus den Polymeren, die aus der Vernetzung der linearen Polymeren, die in Wasser unlöslich sind, wie sie in Anspruch 2 definiert sind, hervorgegangen sind.

7. Injizierbare gelierende Zusammensetzung gemäß einem der Ansprüche 1 bis 5, **dadurch gekennzeichnet, dass** das hydrophile, vernetzte Polymer oder die hydrophilen, vernetzten Polymere aus den Polymeren ausgewählt ist/ sind, die aus der Vernetzung der linearen Polymeren, die in Wasser löslich sind, hervorgegangen sind.

8. Injizierbare gelierende Zusammensetzung gemäß Anspruch 7, **dadurch gekennzeichnet, dass** die linearen Polymere, die in Wasser löslich sind, ausgewählt sind aus Alginaten; Stärkederivaten; Celluloseethern; Celluloseacetaten mit einem Substitutionsgrad zwischen 0,6 und 0,8; Cellulosesulfaten; wasserlöslichen Polysacchariden; Chitosansalzen; Acryl- und Methacrylpolymeren; substituierten oder nicht-substituierten Polyacrylamiden und Polymethacrylamiden; hydrolysierten Derivaten von Polyvinylacetaten; Polymeren, die von Polyoxyethylen, Polyethylenimin abgeleitet sind; löslichen Salzen von Polyvinylpyridin; Polyvinylpyrrolidon; Polyurethanen; ihren Salzen und ihren Copolymeren.

9. Injizierbare gelierende Zusammensetzung gemäß Anspruch 6, **dadurch gekennzeichnet, dass** das vernetzte Polymer oder die vernetzten Polymere ausgewählt ist/sind aus vernetzten Polymeren von Hydroxyethylmethacrylat, Hydroxypropylmethacrylat oder Poly(N-acryloyl-2-amino-2-hydroxymethyl-1,3-propandiol) sowie aus vernetzten Copolymeren von Hydroxyethylmethacrylat und Poly(N-acryloyl-2-amino-2-hydroxymethyl-1,3-propandiol) oder Hydroxypropylmethacrylat und Poly(N-acryloyl-2-amino-2-hydroxymethyl-1,3-propandiol).

10. Injizierbare gelierende Zusammensetzung gemäß einem der vorangehenden Ansprüche, **dadurch gekennzeichnet, dass** der Vernetzungsgrad des vernetzten Polymers zwischen 0,5 und 12% (M/V) liegt.

11. Injizierbare gelierende Zusammensetzung gemäß einem der vorangehenden Ansprüche, **dadurch gekennzeichnet, dass** das vernetzte Polymer oder die vernetzten Polymere 1 bis 30% (M/V) ausmachen.

12. Injizierbare gelierende Zusammensetzung gemäß einem der vorangehenden Ansprüche, **dadurch gekennzeichnet, dass** die Größe der Partikel des vernetzten Polymers/der vernetzten Polymere zwischen 1 und 1.000 $\mu$m liegt.

13. Injizierbare gelierende Zusammensetzung gemäß einem der vorangehenden Ansprüche, **dadurch gekennzeichnet, dass** sie wenigstens ein lineares Polymer von Hydroxyethylmethacrylat oder Hydroxypropylmethacrylat oder ein lineares Copolymer auf der Basis von Hydroxyethylmethacrylat oder Hydroxypropylmethacrylat und Partikel aus vernetzten Polymeren von Hydroxyethylmethacrylat, Hydroxypropylmethacrylat oder Poly(N-acryloyl-2-amino-2-hydroxymethyl-1,3-propandiol) und/oder vernetzten Copolymeren von Hydroxyethylmethacrylat und Poly(N-acryloyl-2-amino-2-hydroxymethyl-1,3-propandiol) oder Hydroxypropylmethacrylat und Poly(N-acryloyl-2-amino-2-hydroxymethyl-1,3-propandiol) umfasst.

14. Injizierbare gelierende Zusammensetzung gemäß Anspruch 13, **dadurch gekennzeichnet, dass** sie umfasst:

- wenigstens ein lineares Copolymer auf der Basis von Hydroxyethylmethacrylat oder Hydroxypropylmethacrylat und
- wenigstens Partikel aus vernetzten Copolymeren von Hydroxyethylmethacrylat oder Hydroxypropylmethacrylat und Poly(N-acryloyl-2-amino-2-hydroxymethyl-1,3-propandiol).

15. Injizierbare gelierende Zusammensetzung gemäß einem der vorangehenden Ansprüche, **dadurch gekennzeichnet, dass** das mit Wasser mischbare, biokompatible Lösungsmittel oder die mit Wasser mischbaren, biokompatiblen Lösungsmittel ausgewählt ist/sind aus N-Methylpyrrolidon, Dimethylethylamid, Diethylenglycoldimethylether, Ethylacetat, Ethanol, Dimethoxyethan, Dimethylsulfoxid, Glycofurol und ihren Gemischen.

16. Injizierbare gelierende Zusammensetzung gemäß Anspruch 15, **dadurch gekennzeichnet, dass** die Lösungsmittel aus Ethanol und N-Methylpyrrolidon ausgewählt sind.

**17.** Injizierbare gelierende Zusammensetzung gemäß einem der vorangehenden Ansprüche, **dadurch gekennzeichnet, dass** sie außerdem ein Adjuvans oder mehrere Adjuvantien, ausgewählt aus Färbemitteln; Markern für bildgebende Untersuchung; antiinflammatorischen Mitteln; angiogenen Mitteln; Antimitotika; Angiogeneseinhibitoren; Wachstumsfaktoren; Vitaminen; Hormonen; Proteinen; Vakzinen; Peptiden; Antiseptika und antimikrobiellen Mitteln, umfasst.

**18.** Injizierbare gelierende Zusammensetzung, wie sie in einem der Ansprüche 1 bis 17 definiert ist, zur Verwendung beim Auffüllen von Kanälen und Kavitäten.

**19.** Injizierbare gelierende Zusammensetzung, wie sie in einem der Ansprüche 1 bis 17 definiert ist, zur Verwendung für die Durchführung von therapeutischen Gefäßokklusionen, zur Geweberekonstruktion, Behandlung von gastroösophagealem Reflux oder Harninkontinenz, zur perkutanen Implantation oder zur Faltenverringerung.

**20.** Intermediärlösung auf der Basis von vernetztem Copolymer, **dadurch gekennzeichnet, dass** sie umfasst:

- wenigstens ein lineares Copolymer von Hydroxypropylmethacrylat und Acrylnitril und/oder wenigstens ein Copolymer von Hydroxypropylmethacrylat und N-tert-Butylacrylamid und/oder wenigstens ein Copolymer von Hydroxypropylmethacrylat und Acetoacetoxyethylmethacrylat und
- wenigstens ein mit Wasser mischbares, biokompatibles Lösungsmittel;

wobei, wenn die genannte Intermediärlösung ein lineares Copolymer von Hydroxypropylmethacrylat und Acrylnitril umfasst, das Lösungsmittel selbstverständlich nicht Dimethylsulfoxid oder Dimethylformamid ist.

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5580568 A
- US 5695480 A
- US 5851508 A

**Littérature non-brevet citée dans la description**

- **Mottu F et al.** *PDA J. Pharm. Sci. Technol.,* 2000, vol. 54 (6), 456-469
- Polymer Science Dictionnary, Essex (England). Elsevier Science Publishers, 1989